(19) Europäisches Patentamt
European Patent Office
Office européen des brevets

(11) EP 2 123 272 A1

(12) **EUROPEAN PATENT APPLICATION**
published in accordance with Art. 153(4) EPC

(43) Date of publication:
**25.11.2009 Bulletin 2009/48**

(21) Application number: **07860343.8**

(22) Date of filing: **27.12.2007**

(51) Int Cl.:
***A61K 31/375*** *(2006.01)* ***A61K 8/49*** *(2006.01)*
***A61K 8/67*** *(2006.01)* ***A61P 17/00*** *(2006.01)*
***A61P 17/16*** *(2006.01)* ***A61P 25/28*** *(2006.01)*
***A61P 29/00*** *(2006.01)* ***A61P 35/00*** *(2006.01)*
***A61P 43/00*** *(2006.01)* ***A61Q 19/08*** *(2006.01)*
***C07D 307/62*** *(2006.01)*

(86) International application number:
**PCT/JP2007/075121**

(87) International publication number:
**WO 2008/090717 (31.07.2008 Gazette 2008/31)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **26.01.2007 JP 2007016536**

(71) Applicant: **Shiseido Company, Ltd.**
**Chuo-ku**
**Tokyo 104-8010 (JP)**

(72) Inventors:
- **HIRUMA, Takuya**
**Yokohama-shi**
**Kanagawa 224-8558 (JP)**
- **FUKUNISHI, Hirotada**
**Yokohama-shi**
**Kanagawa 224-8558 (JP)**
- **SUETSUGU, Masaru**
**Yokohama-shi**
**Kanagawa 224-8558 (JP)**
- **MATSUNAGA, Yukiko**
**Yokohama-shi**
**Kanagawa 236-8643 (JP)**
- **AMANO, Satoshi**
**Yokohama-shi**
**Kanagawa 236-8643 (JP)**
- **SHIBATA, Michio**
**Yokohama-shi**
**Kanagawa 236-8643 (JP)**

(74) Representative: **Adam, Holger et al**
**Kraus & Weisert**
**Patent- und Rechtsanwälte**
**Thomas-Wimmer-Ring 15**
**80539 München (DE)**

# (54) ADAM INHIBITOR

(57) Disclosed is an ADAM inhibitor which enables to prevent or improve conditions caused by increase in ADAM activity. Specifically disclosed is an ascorbic acid derivative represented by the general formula (1) below or a salt thereof. [chemical formula 1] (1), wherein R represents a linear or branched alkyl group having 1 to 22 carbon atoms.

EP 2 123 272 A1

## Description

FIELD OF THE INVENTION

The present invention relates to a novel ADAM inhibitor.

BACKGROUND ART

A group of the membrane type metalloproteases called ADAM (a disintegrin and metalloprotease) are enzymes which play important roles in interactions between cells and extracellular matrices and known to be greatly involved in the differentiation and proliferation of cells, extension and myelination of axons and the like (see, for example, Non-Patent Reference 1). In addition, ADAM as well as the matrix metalloprotease (MMP) has a function of cutting various growth factors including heparin-binding EGF-like growth factor (HB-EGF) and tumor necrosis factor (TNF), transforming growth factor; TGF, cytokines, amyloid precursor protein (APP) and many other membrane proteins on the cell surface and releasing them out of the cell. It is known that ADAM is highly expressed in the pathologic tissues of various malignant tumors (glioma, lung cancer, breast cancer, etc.), rheumatism, and in the cerebrum of patients with Alzheimer's disease, and promotes proliferation and infiltration of the cell or induces the angiogenesis essential for infiltration of cancer cells (see,for example, Non-Patent References 2-6). Therefore, suppression of ADAM activity is considered to be very important in preventing progression of the disease such as cancer, rheumatism, Alzheimer's disease and the like.

Non-Patent Reference 1: Yang P, Baker KA, Hagg T. The ADAMs family: Coordinators of nervous system development, plasticity and repair. Prog Neurobiol 2006; 79: 73-94.
Non-Patent Reference 2: Arribas J, Bech-Serra JJ, Santiago-Josefat B. ADAMs, cell migration and cancer. Cancer Metastasis Rev 2006; 25: 57-68.
Non-Patent Reference 3: Kodama T, Ikeda E, Okada A et al. ADAM12 is selectively overexpressed in human glioblastomas and is associated with glioblastoma cell proliferation and shedding of heparin-binding epidermal growth factor. Am J Pathol 2004; 165: 1743-53.
Non-Patent Reference 4: Liu PC, Liu X, Li Y et al. Identification of ADAM10 as a Major Source of HER2 Ectodomain Sheddase Activity in HER2 Overexpressing Breast Cancer Cells. Cancer Biol Ther 2006; 5: 657-64.
Non-Patent Reference 5: Rocks N, Paulissen G, Quesada Calvo F et al. Expression of a disintegrin and metalloprotease (ADAM and ADAMTS) enzymes in human non-small-cell lung carcinomas (NSCLC). Br J Cancer 2006; 94: 724-30.
Non-Patent Reference 6: Asakura, M. et al. 2002, Cardiac hypertrophy is inhibited by antagonism of ADAM12 processing of HB-EGF: metalloproteinase inhibitors as a new therapy, Nat. Med. 8: 35-40.

DISCLOSURE OF THE INVENTION

PROBLEMS TO BE SOLVED BY THE INVENTION

WhilTAPI-1{N-(R)-(2-(Hydroxyaminocarbonyl)methyl)-4-Methylpentano yl-L-Nal-L-Alanine2-Aminoethyl amide(L-Nal:L-3-(2'-Naphthyl)alanine)} and TAPI-2{N-(R)-(2-(Hydroxyaminocarbonyl)methyl)-4-Methylpentanoyl-L-t-Butyl-Gl ycyl-L- Alanine2-Aminoethyl amide} are known as ADAM inhibitors, ADAM inhibitory ability of those substances is not sufficient and their safety as external medications has not been established. Therefore, there has been a need for developing a safer and more efficacious agent.

The present invention has been attained in view of the above situation and for the purpose of providing an ADAM inhibitor, which has an ADAM inhibiting action and can prevent or ameliorate the diseases caused by increase of ADAM activity.

MEANS FOR SOLVING THE PROBLEMS

ADAM is an enzyme which exists on the cell surface, and it releases the growth factors including HB-EGF, TNF-α, TGF-α, and cytokines, amyloid - precursors, etc. out of the cell membrane, and activates them. One of the important mechanisms for induction or aggravation of diseases including cancers, rheumatism or Alzheimer's disease is considered that ADAM is activated or its expression is enhanced by the signal of malignant transformation or external stimuli leading to promotion of release/activation of the growth factors such as HB-EGF and cytokines as well as differentiation, proliferation and migration of the cell. Therefore, it is assumed that induction or progression of the conditions such as cancers, rheumatism or Alzheimer's disease could be prevented or ameliorated by suppressing ADAM activity

leading to suppressing differentiation, proliferation or filtration of the cell. Further, the invention could not only be applied to the diseases caused by increase of ADAM activity but also could prevent or ameliorate the wrinkle by application to the wrinkle caused by hyperplasia of the epidermal cell.

In searching for a new compound which may inhibit ADAM, the inventors found that certain ascorbic acid derivatives and salts thereof have high ADAM inhibitory activity, thus leading to attainment of the invention.

The invention is an ADAM inhibitor characterized by comprising an ascorbic acid derivative represented by the general formula (1) described below or a salt thereof:

[Formula 1]

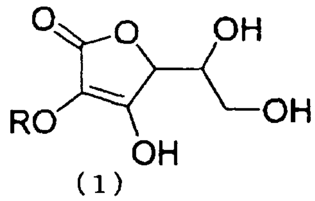

(1)

wherein, R represents a linear or branched alkyl group having 1 to 22 carbon atoms.

The ascorbic acid derivatives of the invention are those which prevent abnormal differentiation, proliferation and migration of the cell by inhibiting or suppressing ADAM activity.

Further, the invention provides a method for inhibiting ADAM **characterized in that** the ADAM inhibitor is applied to the skin to prevent or ameliorate the conditions caused by increase of ADAM activity.

EFFECT OF THE INVENTION

The ADAM inhibitor of the invention can inhibit ADAM activity present in the tissue to suppress very effectively the release/activation of different growth factors and cytokines on the cell surface as well as differentiation, proliferation and migration of the cell.

According to the method of the invention for inhibiting ADAM, the conditions caused by increase of ADAM activity can be prevented or ameliorated effectively using an ADAM inhibitor described above.

BEST MODE FOR CARRYING OUT THE INVENTION

The most preferred embodiments of the invention will be described below. An ascorbic acid derivative or a salt thereof used in the invention is represented as general formula (1) described below.

[Formula 2]

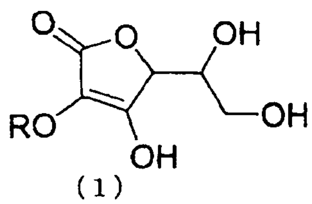

(1)

wherein, R represents a linear or branched alkyl group having 1 to 22 carbon atoms.

R in the ascorbic acid derivative (1) of the inventive compounds is a linear or branched alkyl group having 1 to 22 carbon atoms, preferably a linear or branched alkyl group having 4 to 18 carbon atoms, more preferably a linear or branched alkyl group having 12 to 18 carbon atoms. Such groups include, for example, methyl, ethyl, propyl, isopropyl, butyl, isobutyl, tert-butyl, pentyl, hexyl, heptyl, octyl, 2-ethylhexyl, nonyl, decyl, undecyl, dodecyl, tridecyl, tetradecyl, pentadecyl, hexadecyl, heptadecyl, octadecyl, nonadecyl, eicosane, heneicosane, docosane, etc., preferably, butyl, isobutyl, tert-butyl, pentyl, hexyl, heptyl, octyl, 2-ethylhexyl, nonyl, decyl, undecyl, dodecyl, tridecyl, tetradecyl, pentadecyl, hexadecyl, heptadecyl, octadecyl, etc., more preferably, dodecyl, tridecyl, tetradecyl, pentadecyl, hexadecyl, heptadecyl, octadecyl, etc.

Ascorbic acid derivatives of the invention include, for example, 2-O-methylascorbic acid, 2-O-ethylascorbic acid, 2-O-propylascorbic acid, 2-O-isopropylascorbic acid, 2-O-butylascorbic acid, 2-O-isobutylascorbic acid, 2-O-tert-butylascorbic acid, 2-O-pentylascorbic acid, 2-O-hexylascorbic acid, 2-O-heptylascorbic acid, 2-O-octylascorbic acid, 2-O-(2-ethylhexyl)ascorbic acid, 2-O-nonylascorbic acid, 2-O-decylascorbic acid, 2-O-undecylascorbic acid, 2-O-dodecylascorbic acid, 2-O-tridecylascorbic acid, 2-O-tetradecylascorbic acid, 2-O-pentadecylascorbic acid, 2-O-hexadecylascorbic acid, 2-O-heptadecylascorbic acid, 2-O-octadecylascorbic acid, 2-O-nonadecylascorbic acid, 2-O-eicosane ascorbic acid, 2-O-heneicosane ascorbic acid, 2-O-docosane ascorbic acid, etc. Among them, 2-O-dodecylascorbic acid and 2-O-octadecylascorbic acid are particularly preferable.

Ascorbic acid derivatives used in the invention include, besides free acids, medically acceptable salt forms thereof. These salts include, for example, but not limited to, alkali metal salts such as sodium and potassium salts, alkaline earth metal salts such as calcium and magnesium salts, amine salts such as methylamine salts, dimethylamine salts, trimethylamine salts, methylpiperidine salts, ethanolamine salts, diethanolamine salts, triethanolamine salts and lysine salts, ammonium salts or basic amino acid salts.

Ascorbic acid derivatives and salts thereof may be produced according to the known producing methods.

The ADAM inhibitor comprising an ascorbic acid derivative or a salt thereof may be used as but not limited to dermatological external agents, powders, granules, ampules and injections.

In case of using an ADAM inhibitor of the invention in a dermatological external agent, the combination amount of an ascorbic acid derivative or a salt thereof varies depending on the its use aspect and product form, being, for example, but not particularly limited to preferably 0.001-10% by mass, more preferably 0.005-5% by mass, even more preferably 0.01-1% by mass based on the total amount.

In the specification here, the "dermatological external agents" include cosmetics, drugs and quasi drugs, etc. Its formulations include any such as an aqueous, solubilizing, emulsified, oil, gel, paste, ointment, aerosol, water-oil bilayer and water-oil-powder three layer systems. They also include those carried on the sheeted base.

The dermatological external agents can also adopt any product forms and uses. For example, it can be used as an external agent for the face, body or scalp including lotions, emulsions, creams and packs.

The dermatological external agent may mix properly as needed besides the above described ascorbic acid derivative or a salt thereof, any other components used in the dermatological external agent including ordinary cosmetics and drugs, and produced according to the conventional methods depending on the intended formulation. For example, an ascorbic acid derivative or a salt thereof may be mixed with one or two or more of the ingredients below to prepare a dermatological external agent.

Ultraviolet light absorbing agents include, for example, ultraviolet absorber of benzoic acid system such as para-aminobenzoic acid (hereinafter, abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA methyl ester, etc.; ultraviolet absorber of anthranilic acid system such as homomenthyl-N-acetylanthranilate; ultraviolet absorber of salicylic acid system such as amyl salicylate, menthyl salicylate, homomenthyl salicylate, octyl salicylate, phenyl salicylate, benzyl salicylate, p-isopropanol phenyl salicylate, etc.; ultraviolet absorber of cinnamic acid system such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-$\alpha$-cyano-$\beta$-phenyl cinnamate, 2-ethylhexyl-$\alpha$-cyano-$\beta$-phenyl cinnamate, glycerylmono-2-ethylhexanoyl-dipara-methoxy cinnamate, and methyl bis(trimethylsiloxane)silylisopentyl trimethoxy cinnamate, etc.; 3-(4'-methylbenzylidene)-d,1-camphor; 3-benzylidene-d,1-camphor; urocanic acid; urocanic acid ethyl ester, 2-phenyl-5-methylbenzoxazole, 2,2'-hydroxy-5-methylphenylbenzotriazole; 2-(2'-hydroxy-5'-t-octylphenyl)benzotriazole; 2-(2'-hydroxy-5'-methylphenylbenzotriazole; dibenzaladine, dianisoylmethane, 4-methoxy-4'-t-butyldibenzoylmethane; 5-(3,3-dimethyl-2-norbornylidene)-3-pentane-2-one; dimorpholinopyridazinone, etc. and any one or two or more may be used.

Ultraviolet light scattering agents include, for example, powders such as titanium oxide, particulate titanium oxide, zinc oxide, particulate zinc oxide, ferric oxide, particulate ferric oxide, and ceric oxide, etc.

For these ultraviolet light scattering agent, a spicular, spindle, spherical or granular powder is usually used. In addition, particulate powders which have the particle size of not more than 0.1 $\mu$m is preferable.

Also preferable are ultraviolet light scattering agents silicone-treated by methyl hydrogen polysiloxane or silane coupling agent; metallic soap-treated; fluorine-treated by perfluoroalkyl phosphate diethanolamine salt or perfluoroalkyl silane; hydrophobilized-treated by dextrin fatty acid ester treatment and the like.

Liquid oils include, for example, avocado oil, camellia oil, turtle oil, macadamia nuts oil, corn oil, mink oil, olive oil, rape seed oil, yolk oil, sesame oil, persic oil, wheat embryo oil, sasanqua oil, castor oil, flaxseed oil, safflower oil,

cottonseed oil, perilla oil, soybean oil, peanut oil, tea seed oil, kaya oil, rice bran oil, shinagiri oil, Japan tung oil, jojoba oil, embryo oil, triglycerin, and the like.

Solid oils include, for example, cocoa butter, coconut oil, equine tallow, hydrogenated coconut oil, palm oil, beef tallow, mutton tallow, hydrogenated beef oil, palm kernel oil, lard, bovine bone tallow, Japan kernel oil, hydrogenated oil, bovine leg tallow, Japan wax, hydrogenated castor oil, and the like.

Waxes include, for example, beeswax, candelilla wax, cotton wax, carnauba wax, baybery wax, ibota wax, spermaceti wax, montan wax, rice wax, lanolin, kapok wax, lanolin acetate, liquid lanolin, sugarcane wax, lanolin fatty acid isopropyl, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, POE hydrogenated lanolin alcohol ether, and the like.

Hydrocarbon oils include, for example, liquid paraffin, ozokerite, squalane, pristane, paraffin, ceresin, squalene, vaseline, microcrystalline wax, polyethylene wax, Fischer-Tropsch wax, etc.

Higher fatty acids include, for example, lauric acid, myristic acid, palmitic acid, stearic acid, behenic acid, oleic acid, undecylenic acid, tolic acid, linoleic acid, linolenic acid, eicosapentaenoic acid (EPA), docosahexaenoic acid (DHA), etc.

Higher alcohols include, for example, straight chain alcohols (e.g., lauryl alcohol, cetyl alcohol, stearyl alcohol, behenyl alcohol, myristyl alcohol, oleyl alcohol, cetostearyl alcohol, etc.); branched chain alcohols (e.g., monostearyl glycerin ether (batyl alcohol), 2-decyltetradecynol, lanolin alcohol, cholesterol, phytosterol, hexyldodecanol, octyldodecanol, etc.) and the like.

Synthesized ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glycerin di-2-heptylundecanoate, trimethylolpropane tri-2-ethyl-hexanoate, trimethylolpropane triisostearate, pentaerythritol tetra-2-ethylhexanoate, glycerin tri-2-ethylhexanoate, glycerin trioctanoate, glycerin triisopalmitate, trimethylolpropane triisostearate, cetyl 2-ethylhexanoate, 2-ethylhexyl palmitate, glycerin trimyristate, glyceride tri-2-heptylundecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, 2-ethylhexyl succinate, triethyl citrate, polyoxyethylene-polyoxypropylene random polymer methyl ether, etc.

Silicone oils include, for example, chain polysiloxanes (e.g., dimethyl polysiloxane, methylphenylpolysiloxane, diphenylpolysiloxane, etc.); cyclic polysiloxanes (e.g., octamethylcyclotetrasiloxane, decamethylcyclopentasiloxane, dodecamethylcyclohexasiloxane, etc.), silicone resins which form three dimensional network structures, silicone gums, a wide variety of denatured polysiloxanes (amino-denatured polysiloxane, polyether-denatured polysiloxane, alkyl-denatured polysiloxane, fluorine-denatured polysiloxane, etc.) and the like.

Others include, for example, humectants such as polyethylene glycol, glycerin, 1,3-butyleneglycol, erythritol, sorbitol, xylitol, maltitol; thickeners such as cellulose, hydroxyethylcellulose, hydroxypropylcellulose, methylhydroxypropylcellulose, methylcellulose, carboxymethylcellulose, quinceseed, carrageenan, pectin, mannan, curdlan, chondroitin sulfate, starch, galactan, dermatan sulfate, glycogen, acacia gum, heparan sulfate, hyaluronic acid, sodium hyaluronate, tragacanth gum, keratan sulfate, chondroitin, xanthan gum, mucoitin sulfate, hydroxyethyl guar gum, carboxymethyl guar gum, guar gum, dextran, kerato sulfate, locust bean gum, succinoglucan, caronic acid, chitin, chitosan, carboxymethylchitin and agar, etc.; lower alcohols such as ethanol; antioxidants such as butylhydroxytoluene, tocopherol, and phytin; antibacterial agents such as benzoic acid, salicylic acid, sorbic acid, paraoxybenzoic acid alkyl ester, and hexachlorophene; organic acids such as acyl sarcosine (e.g., sodium lauloyl sarcosine), glutathione, citric acid, malic acid, tartaric acid and lactic acid; vitamin A and its derivatives, vitamin Bs such as vitamin B6 hydrochloride, vitamin B6 tripalmitate, vitamin B6 dioctanoate, vitamin B2 and its derivatives, vitamin B12, and vitamin B15 and its derivatives, vitamin Cs such as ascorbic acid, ascorbic acid sulfate ester (salt), ascorbic acid phosphate ester (salt), and ascorbic acid dipalmitate, vitamin Es such as $\alpha$-tocopherol, $\beta$-tocopherol, $\delta$-tocopherol, and vitamin E acetate; vitamins such as vitamin Ds, vitamin H, pantothenic acid, and pantethine; saponins such as nicotinamide, benzyl nicotinate, $\gamma$-orizanol, allantoin, glycyrrhizic acid (salt), glycyrrhetic acid and its derivatives, hinokitiol, bisabolol, eucalyptone, thymol, inositol, saikosaponin, ginseng saponin, Luffa cylindrica saponin, and soapberry saponin; various drugs such as pantothenylethyl ether, ethynylestradiol, tranexamic acid, arbutin, cepharanthin, and placenta extract, plant extracts such as Rumex japonicus, Clara, Nuphar japonicum DC, orange, sage, Achillea alpina, Malva sylvestris, Swertia japonica, thyme, Japanese angelica, bitter orange peel, birch, Field Horsetail, Luffa cylindrica, common horse-chestnut, Saxifragaceae, arnica, lily, sagebrush, paeoniae radix, aloe, Gardenia jasminoides, Sawara cypress, Hawthorn extract, Hypericum perforatum extract, iris-inextract, Gambir extract, ginkgo leaf extract, ibukijyakou extract, Foeniculum vulgare extract, Oolong tea extract, water-lily extract, Rosae Multiflorae Fructus extract, Plectranthus japonicus extract, Scutellariae Radix extract, Phellodendri Cortex extract, Lamium album extract, G. uralensis extract, Gardenia jasminoides extract, black tea extract, Tamarix chinensis Lour

extract, Potentilla Erecta extract, rose extract, Luffa cylindrica extract, peppermint extract, rosemary extract, and royal jelly extract, pigment; non-ionic surfactants such as sorbitan monolaurate, sorbitan monopalmitate, sorbitan sesquioleate, sorbitan trioleate, polyoxyethylene sorbitan monolaurate, polyoxyethylene sorbitan monostearate, polyethylene glycol monooleate, polyoxyethylene alkyl ether, polyglycol diether, lauroyldiethanol amide, fatty acid isopropanolamide, maltitolhydroxy fatty acid ether, alkylated polysaccharide, alkyl glucoside, and sugar ester, cationic surfactants such as stearyltrimethylammonium chloride, benzalkonium chloride, and laurylamine oxide, anionic surfactants such as sodium palmitate, sodium laurate, sodium lauryl acid, potassium lauryl sulfate, alkylsulfuric acid triethanolamine ether, turkey-red oil, linear dodecylbenzene sulfate, polyoxyethylene hydrogenated castor oil maleic acid, and acylmethyl taurine; ampholytic surfactants; neutralizers; antioxidants such as δ-tocopherol and butylhydroxytoluene, and preservatives such as phenoxyethanol and paraben.

The above described ingredients are only illustrative and the invention is not limited to them. Additionally, these ingredients may be mixed being combined appropriately according to description depending on the desired form.

The ADAM inhibitor of the invention may be used as a drug intended to prevent or ameliorate the conditions caused by increase of ADAM activity.

In order to formulate the ADAM inhibitor of the invention in such uses, for example, powders, granules, ampoules, injections, isotonic solutions and the like are prepared according to ordinary methods. In the case of preparing oral solid preparations, added are excipients and if needed binders, wetting agents, disintegrators, surfactants, lubricants, dispersing agents, flavors and correctives followed by preparation as tablets, coated tablets, granules, capsules or the like according to conventional methods.

The excipients used include, for example, lactose, glucose, sorbitol, cornstarch and mannitol, etc.; the binders include, for example, polyvinyl alcohol, polyvinyl ether, ethylcellulose, acacia gum, gelatin, hydroxypropylcellulose and polyvinylpyrrolidone, etc; the disintegrators include calcium carbonate, calcium citrate, dextrin, starch , gelatin powder, etc.; the lubricants include calcium carbonate, calcium citrate, talc, polyethyleneglycol, etc.; the coloring agents include cocoa powder, mint flavoring acid and mint oil, etc. These tablets and granules may be coated properly if needed by sugar, gelatin and others.

In the case of preparing the injections, added are if needed pH adjusting agents, buffers, surfactants, solubilizers, solvents, stabilizers, preservatives and the like and prepared as subcutaneous, intramuscular or intravenous injections.

In the method of the invention for inhibiting ADAM, the ascorbic acid derivatives or salts thereof may be applied in any forms singly or in combination with any other ingredients as long as they can be applied to the skin and can attain the purpose of the invention. In addition, the location of the skin where they are applied to is not limited but includes the skin of all over the body surface including the scalp. In addition, they may be applied by oral administration or by injection.

Further, use of the ADAM inhibitor comprising an ascorbic acid derivative or a salt thereof is not limited to treatment or prevention of different diseases adversely affected by growth or differentiation of the skin cell due to the activation of ADAM as described above, but includes any uses which can exert the effect by inhibiting the ADAM activity. The invention for example, may not only be applied to the diseases caused by increase of the ADAM activity, but also applied to the wrinkle caused by hyperplasia of the epidermal cells thereby the wrinkle may be prevented or ameliorated.

## EXAMPLES

Examples will now be given to describe the present invention in more detail. The invention is not intended to be limited to them. The combination amounts herein are % by mass.

### 1. Exploring a new ADAM inhibitor

For the ADAM inhibiting effect, the TNF-α which is cut out to be released from the membrane of the U937 cell derived from the human lymphoma through the action of ADAM was used as an index, and the inhibiting effect on the TNF-α release by the agents was assessed. A new ADAM inhibitor was explored by screening different compounds according to the present method.

The U937 cells derived from the human lymphoma were seeded to the 48-well plate adjusting the number of the cells to $1 \times 10^5$ cells/0.25 ml and cultured at 37°C overnight. The medium which contained 5 μg/ml or 30 μg/ml of an ascorbic acid derivative was added and incubated at 37°C for 30 min. to be pretreated. 10 nM PMA (phorbol ester: 12-O-tetradecanoylphorbol-acetate; Sigma P8139) was then added and cultured at 37°C for 6 hours. After completion of the treatment, the supernatant of the culture was collected, centrifuged and the cell-free supernatant was cryopreserved at -20°C. The assay of the released hTNF-α was performed using the hTNF-α Duo-Set ELISA kit from R & D Ltd. Each supernatant of the culture was diluted 5-folds with the medium, then 100 μl each was added to the 96-well plate and measured according to the protocol of R & D Ltd. The absorbance at 450-570 nm of each well was measured by the measurement kit and concentrations of the samples were obtained from the standard curve. The inhibition rates were calculated according to the following equation wherein A0 is the absorbance of 0% inhibiting control (the medium containing only PMA), A100 is the absorbance of 100% inhibiting control (only the medium) and AS is the absorbance of the sample.

$$\text{The inhibition rate (\%)} = (A0 - AS) / (A0 - A100) \times 100$$

Consequently, a high suppressing effect on HB-EGF release was noted for 2-O-dodecyl ascorbic acid and 2-O-octadecyl ascorbic acid which have particularly greater number of carbon atoms among the inventive ascorbic acid derivatives suggesting that they inhibit the ADAM activity. The release suppressing rate of each ascorbic acid derivative is shown in Table 1.

[Table 1]

| Substance tested | Inhibition rate (%) | |
|---|---|---|
| | Concentration | |
| | 5 μg/mL | 30 μg/mL |
| 2-0-dodecyl ascorbic acid | 18.25 | 60.07 |
| 2-0-octadecyl ascorbic acid | 15.19 | 82.70 |

Examples of preparations containing ascorbic acid derivatives or salts thererof according to the invention will be shown below, but the invention is not to be limited to them.

Preparation Example 1: injection

| (Formulation) | % by mass |
|---|---|
| 2-O-dodecyl ascorbic acid | 100 mg |
| Chloroform | 5 ml |
| Tween-80 | 100 mg |
| Sterile distilled water | 10 ml |

(Procedure)

100 mg of finely powdered 2-O-dodecyl ascorbic acid was dissolved in 5 ml of chloroform. 10 mg of Tween-80 was added and mixed, then filtrated through a millipore filter followed by evaporation to dryness with a sterile evaporator. 10 ml of sterile distilled water was added and mixed vigorously, then sonicated and filled into an ampoule to prepare an injection.

Preparation Example 2: emulsion

| (Formulation) | % by mass |
|---|---|
| Stearic acid | 2.5 |
| Cetyl alcohol | 1.5 |
| Vaseline | 5.0 |
| Liquid paraffin | 5.0 |
| Polyoxyethylene (10 mol) monooleate ester | 2.0 |
| Polyethylene glycol 1500 | 3.0 |
| Triethanolamine | 1.0 |
| Carboxy vinyl polymer | 0.05 |
| 2-O-dodecylascorbic acid | 2.0 |
| Sodium bisulfite | 0.01 |
| Ethylparaben | 0.2 |
| Flavor | q.s. |
| Purified water | balance |

(Procedure)

Carboxy vinyl polymer is dissolved in a little amount of the purified water (phase A). Polyethylene glycol 1500, triethanolamine and 2-O-dodecylascorbic acid are added to the remaining purified water, dissolved with heating, then maintained at 70°C (aqueous phase). Other ingredients are mixed, melted with heating, then maintained at 70°C (oil phase). The oil phase is added to the aqueous phase to pre-emulsify, then phase A is added to emulsify homogeneously with a homomixer. After emulsification, it is cooled to 30°C while mixing well to obtain an emulsion.
Preparation Example 3: lotion

| (Formulation) | % by mass |
|---|---|
| Ethanol | 5.0 |
| Carboxy vinyl polymer | 0.3 |
| Polyoxyethylene (15 mol) oleyl ether | 0.8 |
| 1,3-Butylene glycol | 5.0 |
| 2-O-octadecylascorbic acid | 2.0 |
| Citric acid | 0.03 |
| Sodium citrate | 0.07 |
| Methylparaben | 0.1 |
| Purified water | balance |

(Procedure)

Citric acid, sodium citrate and 2-O-octadecylascorbic acid were dissolved in the purified water to make an aqueous phase. On the other hand, other ingredients were dissolved with stirring. This was added to the aqueous phase, and made homogeneous to obtain a lotion.
Preparation Example 4: cream

| (Formulation) | % by mass |
|---|---|
| Stearic acid | 2.0 |
| Stearyl alcohol | 7.0 |
| Hydrous lanolin | 2.0 |
| 2-Octyldodecylalcohol | 6.0 |
| Polyoxyethylene (25 mol) cetyl alcohol ether | 3.0 |
| Glycerin monostearate ester | 2.0 |
| 2-O-dodecylascorbic acid | 2.0 |
| 2-O-octadecylascorbic acid | 2.0 |
| 1,3-Butylene glycol | 5.0 |
| Trisodium edetate | 0.1 |
| Sodium glycyrrhetinate | 0.1 |
| Vitamin E acetate | 0.3 |
| Ethylparaben | 0.3 |
| Purified water | balance |

(Procedure)

2-O-dodecylascorbic acid, 2-O-octadecylascorbic acid, 1,3-butylene glycol, trisodium edetate and sodium glycyrrhetinate were added to the purified water and maintained at 70°C to make an aqueous phase. On the other hand, other ingredients were heated with stirring, and melted then maintained at 70°C to make an oil phase. The oil phase was added to the aqueous phase to pre-emulsify, then emulsified homogenously with a homomixer followed by cooling to 30°C to obtain a cream.
Preparation Example 5: emulsion

| (Formulation) | % by mass |
|---|---|
| Dimethyl polysiloxane | 3.0 |

(continued)

| (Formulation) | % by mass |
|---|---|
| Decamethylcyclopenta siloxane | 4.0 |
| Ethanol | 5.0 |
| Glycerin | 6.0 |
| 1,3-Butylene glycol | 5.0 |
| 2-O-dodecylascorbic acid | 0.5 |
| 2-O-octadecylascorbic acid | 0.5 |
| Polyoxyethylene methyl glucoside | 3.0 |
| Sunflower oil | 1.0 |
| Squalene | 2.0 |
| Potassium hydroxide | 0.1 |
| Sodium hexametaphosphate | 0.05 |
| Hydoxypropyl-β-cyclodextrin | 0.1 |
| Potassium 4-methoxysalicilate | 1.0 |
| Dipotassium glycyrrhizinate | 0.05 |
| Loquat leaf extract | 0.1 |
| Sodium L-glutamate | 0.05 |
| Fennel extract | 0.1 |
| Yeast extract | 0.1 |
| Lavender oil | 0.1 |
| Rehrnannia root extract | 0.1 |
| Dimorphorinopyridazinone | 0.1 |
| Xanthan gum | 0.1 |
| Carboxy vinyl polymer | 0.1 |
| Alkyl acrylate/methacrylate copolymer (PemulenTR-1) | 0.1 |
| Colcothar | q.s. |
| Yellow iron oxide | q.s. |
| Paraben | q.s. |
| Purified water | balance |

Preparation Example 6: emulsion

| (Formulation) | % by mass |
|---|---|
| Dimethyl polysiloxane | 3.0 |
| Decamethylcyclopentasiloxane | 4.0 |
| Ethanol | 5.0 |
| Glycerin | 6.0 |
| 1,3-Butylene glycol | 5.0 |
| 2-O-octadecylascorbic acid | 1.0 |
| Polyoxyethylene methyl glucoside | 3.0 |
| Sunflower oil | 1.0 |
| Squalene | 2.0 |
| Potassium hydroxide | 0.1 |
| Sodium hexametaphosphate | 0.05 |
| Hydoxypropyl-β-cyclodextrin | 0.1 |
| Potassium 4-methoxysalicilate | 1.0 |
| Dipotassium glycyrrhizinate | 0.05 |
| Loquat leaf extract | 0.1 |
| Sodium L-glutamate | 0.05 |
| Fennel extract | 0.1 |
| Yeast extract | 0.1 |

(continued)

| (Formulation) | | % by mass |
|---|---|---|
| Lavender oil | | 0.1 |
| Rehrnannia root extract | | 0.1 |
| Dimorpholinopyridazinone | | 0.1 |
| Xanthan gum | | 0.1 |
| Carboxy vinyl polymer | | 0.1 |
| Alkyl acrylate/methacrylate copolymer (PemulenTR-1) | | 0.1 |
| Colcothar | | q.s. |
| Yellow iron oxide | | q.s. |
| Paraben | ■ BR>@ | q.s. |
| Purified water | | balance |

Preparation Example 7: emulsion

| (Formulation) | % by mass |
|---|---|
| Dimethyl polysiloxane | 2.0 |
| Behenyl alcohol | 1.0 |
| Batyl alcohol | 0.5 |
| Glycerin | 5.0 |
| 1,3-Butylene glycol | 7.0 |
| 2-O-dodecylascorbic acid | 0.5 |
| 2-O-octadecylascorbic acid | 1.0 |
| Tranexamic acid methylamide hydrochloride | 0.7 |
| Erythritol | 2.0 |
| Hydrogenated oil | 3.0 |
| Squalene | 6.0 |
| Pentaerythrit tetra 2-ethylhexanoate | 2.0 |
| Polyoxyethylene glyceryl isostearate | 1.0 |
| Polyoxyethylene glycerin monostearate | 1.0 |
| Potassium hydroxide | q.s |
| Sodium hexametaphosphate | 0.05 |
| Phenoxyethanol | q.s. |
| Carboxy vinyl polymer | 0.1 |
| Purified water | balance |

Preparation Example 8: emulsion

| (Formulation) | % by mass |
|---|---|
| Dimethyl polysiloxane | 2.0 |
| Behenyl alcohol | 1.0 |
| Batyl alcohol | 0.5 |
| Glycerin | 5.0 |
| 1,3-Butylene glycol | 7.0 |
| 2-O-octadecylascorbic acid | 1.0 |
| Tranexamic acid methylamide hydrochloride | 0.7 |
| Erythritol | 2.0 |
| Hydrogenated oil | 3.0 |
| Squalene | 6.0 |
| Pentaerythrit tetra 2-ethylhexanoate | 2.0 |
| Polyoxyethylene glyceryl isostearate | 1.0 |
| Polyoxyethylene glycerin monostearate | 1.0 |

(continued)

| (Formulation) | % by mass |
|---|---|
| Potassium hydroxide | q.s. |
| Sodium hexametaphosphate | 0.05 |
| Phenoxyethanol | q.s. |
| Carboxy vinyl polymer | 0.1 |
| Purified water | balance |

Preparation Example 9: emulsion

| (Formulation) | % by mass |
|---|---|
| Liquid paraffin | 7.0 |
| Vaseline | 3.0 |
| Decamethylcyclopentasiloxane | 2.0 |
| Behenyl alcohol | 0.5 |
| Glycerin | 5.0 |
| Dipropylene glycol | 7.0 |
| 2-O-dodecylascorbic acid | 1.0 |
| 2-O-octadecylascorbic acid | 1.0 |
| Polyethylene glycol 1500 | 2.0 |
| Jojoba oil | 1.0 |
| Isostearic acid | 0.5 |
| Stearic acid | 0.5 |
| Behenic acid | 0.5 |
| Pentaerythrit tetra 2-ethylhexanoate | 3.0 |
| Cetyl 2-ethylhexanoate | 3.0 |
| Glycerin monostearate | 1.0 |
| Polyoxyethylene glycerin monostearate | 1.0 |
| Potassium hydroxide | 0.1 |
| Sodium hexametaphosphate | 0.05 |
| Stearyl glycyrrhetinate | 0.05 |
| L-arginine hydrochloride | 0.1 |
| Royal jelly extract | 0.1 |
| Yeast extract | 0.1 |
| Curcuma extract | 0.1 |
| Tocopherol acetate | 0.1 |
| Acetylated sodium hyaluronate | 0.1 |
| Trisodium edetate | 0.05 |
| 4-t-Butyl-4'-methoxydibenzoylmethan | 0.1 |
| 2-Ethylhexyl Para-methoxycinnamate | 0.1 |
| Carboxy vinyl polymer | 0.15 |
| Paraben | q.s. |
| Purified water | balance |
| Flavor | q.s. |

Preparation Example 10: emulsion

| (Formulation) | % by mass |
|---|---|
| Vaseline | 5.0 |
| Behenyl alcohol | 0.5 |
| Batyl alcohol | 0.5 |
| Glycerin | 7.0 |

(continued)

| (Formulation) | % by mass |
|---|---|
| 1,3-Butylene glycol | 7.0 |
| 2-O-tridecylascorbic acid | 1.0 |
| 2-O-tetradecylascorbic acid | 1.0 |
| 1,2-pentanediol | 1.0 |
| Xylit | 3.0 |
| Polyethylene glycol 20000 | 2.0 |
| Hydrogenated oil | 2.0 |
| Jojoba oil | 2.0 |
| Squalene | 5.0 |
| Isostearic acid | 0.5 |
| Pentaerythrit tetra 2-ethylhexanoate | 2.0 |
| Polyoxyethylene hydrogenated castor oil | 0.5 |
| Lauryldimethylaminoacetic acid betaine | 0.4 |
| Potassium hydroxide | q.s. |
| Sodium pyrosulfite | 0.01 |
| Sodium hexametaphosphate | 0.05 |
| Dipotassium glycyrrhizinate | 0.05 |
| Trimethylglycine | 3.0 |
| Arbutin | 3.0 |
| Pyrola japonica extract | 0.1 |
| Deadnettle extract | 0.1 |
| Lempuyang extract | 0.1 |
| Yeast extract | 0.1 |
| Tocopherol acetate | 0.1 |
| Thiotaurine | 0.1 |
| Clara extract | 0.1 |
| Colcothar | q.s. |
| Qinceseed extract | 0.1 |
| Carboxy vinyl polymer | 0.2 |
| Phenoxyethanol | q.s. |
| Purified water | balance |

Preparation Example 11: emulsion

| (Formulation) | % by mass |
|---|---|
| Vaseline | 5.0 |
| Dimethyl polysiloxane | 2.0 |
| Behenyl alcohol | 0.6 |
| Batyl alcohol | 0.5 |
| Dipropylene glycol | 2.0 |
| 1,3-Butylene glycol | 4.0 |
| 2-O-pentadecylascorbic acid | 10.0 |
| 2-O-octadecylascorbic acid | 3.0 |
| Xylit | 1.0 |
| Polyethylene glycol 1500 | 1.0 |
| Squalene | 5.0 |
| Glyceryl Tri2-ethylhexanoate | 2.0 |
| Retinol acetate | 0.03 |
| Retinol (1.5 million unit) | 0.01 |
| Polyoxyethylene hydrogenated castor oil | 0.5 |

(continued)

| (Formulation) | % by mass |
|---|---|
| Tranexamic acid methylamide hydrochloride | 0.7 |
| Dipotassium glycyrrhizinate | 0.1 |
| 2-O-ethyl-L-ascorbic acid | 0.1 |
| Yeast extract | 0.1 |
| Gambir extract | 0.1 |
| Peony root extract | 0.1 |
| Belamcanda extract | 0.1 |
| Trisodium HEDTA | 0.05 |
| Xanthan gum | 0.1 |
| Carboxy vinyl polymer | 0.15 |
| Purified water | balance |
| Flavor | q.s. |

Preparation Example 12: lotion

| (Formulation) | % by mass |
|---|---|
| Ethylalcohol | 5.0 |
| Glycerin | 1.0 |
| 1,3-Butylene glycol | 5.0 |
| 2-O-octadecylascorbic acid | 3.0 |
| 2-O-hexadecylascorbic acid | 10.0 |
| Polyoxyethylene polyoxypropylene decyl tetradecyl ether | 0.2 |
| Sodium hexametaphosphate | 0.03 |
| Trimethylglycine | 1.0 |
| Sodium polyaspartate | 0.1 |
| α-Tocopherol 2-L-ascorbate diesterpotassium phosphate | 0.1 |
| 2-O-ethyl-L-ascorbic acid | 0.1 |
| Green tea extract | 0.1 |
| Beech extract | 0.1 |
| Peach kernel | 0.1 |
| Peppermint extract | 0.1 |
| Iris root extract | 0.1 |
| Trisodium HEDTA | 0.1 |
| Carboxy vinyl polymer | 0.05 |
| Potassium hydroxide | 0.02 |
| Phenoxyethanol | q.s. |
| Purified water | balance |
| Flavor | q.s. |

Preparation Example 13: lotion

| (Formulation) | % by mass |
|---|---|
| Glycerin | 2.0 |
| 1,3-Butylene glycol | 4.0 |
| 2-O-heptadecylascorbic acid | 1.0 |
| 2-O-octadecylascorbic acid | 1.0 |
| Erythritol | 1.0 |
| Polyoxyethylene methylglucoside | 1.0 |
| Polyoxyethylene hydrogenated castor oil | 0.5 |
| L-ascorbic acid 2-glucoside | 2.0 |

(continued)

| (Formulation) | % by mass |
|---|---|
| Xylitol | 0.1 |
| Citric acid | 0.02 |
| Sodium citric acid | 0.08 |
| Phenoxyethanol | q.s. |
| N-coconut oil fatty acid acyl L-arginine ethyl/DL-pyrrolidone carboxylic acid | 0.1 |
| Purified water | balance |

Preparation Example 14: lotion

| (Formulation) | % by mass |
|---|---|
| Glycerin | 2.0 |
| 1,3-Butylene glycol | 4.0 |
| 2-O-dodecylascorbic acid | 1.0 |
| 2-O-octadecylascorbic acid | 1.0 |
| Erythritol | 1.0 |
| Polyoxyethylene methylglucoside | 1.0 |
| Polyoxyethylene hydrogenated castor oil | 0.5 |
| L-ascorbic acid 2-glucoside | 2.0 |
| Xylitol | 0.1 |
| Citric acid | 0.02 |
| Sodium citric acid | 0.08 |
| Phenoxyethanol | q.s. |
| N-coconut oil fatty acid acyl L-arginine ethyl/DL-pyrrolidone carboxylic acid | 0.1 |
| Purified water | balance |

Preparation Example 15: lotion

| (Formulation) | % by mass |
|---|---|
| Ethanol | 10.0 |
| Dipropylene glycol | 1.0 |
| Sodium 2-O-dodecylascorbate | 1.0 |
| Sodium 2-O-tridecylascorbate | 1.0 |
| Polyethylene glycol 1000 | 1.0 |
| Polyoxyethylene methylglucoside | 1.0 |
| Jojoba oil | 0.01 |
| Glyceryl tri2-ethylhexanate | 0.1 |
| Polyoxyethylene hydrogenated castor oil | 0.2 |
| Polyglyceryl diisostearate | 0.15 |
| Sodium N-stearoil-L-glutamate | 0.1 |
| Citric acid | 0.05 |
| Sodium citric acid | 0.2 |
| Potassium hydroxide | 0.4 |
| Dipotassium glycyrrhizinate | 0.1 |
| Arginine hydrochloride | 0.1 |
| L-ascorbic acid 2-glucoside | 2.0 |
| Scutellaria extract | 0.1 |
| Saxifraga stolonifera extract | 0.1 |
| Deadnettle extract | 0.1 |
| Thyme extract | 0.1 |
| Origanum majorana extract | 0.1 |

(continued)

| (Formulation) | % by mass |
|---|---|
| Tranexamic acid | 1.0 |
| Trisodium edetate | 0.05 |
| 2-Ethylhexyl para-methoxycinnamate | 0.01 |
| Dibutylhydroxytoluene | q.s. |
| Paraben | q.s. |
| Ocean deep water | 3.0 |
| Purified water | balance |
| Flavor | q.s. |

Preparation Example 16: lotion

| (Formulation) | % by mass |
|---|---|
| Dimethyl polysiloxane | 1.0 |
| Ethanol | 3.0 |
| Behenyl alcohol | 0.3 |
| Glycerin | 5.0 |
| Dipropylene glycol | 5.0 |
| 2-O-tetradecylascorbic acid | 0.1 |
| 2-O-octadecylascorbic acid | 0.1 |
| Erythritol | 1.0 |
| Polyethylene glycol 4000 | 1.0 |
| Squalene | 0.4 |
| Cetyl 2-ethylhexanoate | 0.1 |
| Sodium N-stearoyl-L-glutamate | 0.2 |
| Magnesium chloride | 0.1 |
| Arginine chloride | 0.1 |
| Hypotaurine | 0.1 |
| Trisodium edetate | 0.1 |
| Paraben | q.s. |
| Purified water | balance |
| Flavor | q.s. |

Preparation Example 17: lotion

| (Formulation) | % by mass |
|---|---|
| Ethanol | 40.0 |
| Dipropylene glycol | 1.0 |
| 2-O-dodecylascorbic acid | 0.1 |
| 2-O-pentadecylascorbic acid | 0.1 |
| Polyoxyethylene polyoxypropylene decyl tetradecyl ether | 0.1 |
| Anhydrous silicic acid | 1.0 |
| Salicylic acid | 0.1 |
| Sodium citrate | 0.2 |
| Zinc paraphenolsulfonate | 0.2 |
| Dipotassium glycyrrhizinate | 0.1 |
| Pyridoxine hydrochloride | 0.1 |
| L-Serine | 0.1 |
| L-Menthol | 0.05 |
| Trisodium HEDTA | 0.05 |
| Powdered cellulose | 1.0 |

(continued)

| (Formulation) | % by mass |
|---|---|
| Bentonite | 0.8 |
| Purified water | balance |

Preparation Example 18: lotion

| (Formulation) | % by mass |
|---|---|
| Ethanol | 40.0 |
| Dipropylene glycol | 1.0 |
| Potassium 2-O-octadecylascorbate | 0.1 |
| Polyoxyethylene polyoxypropylene decyl tetradecyl ether | 0.1 |
| Anhydrous silicic acid | 1.0 |
| Salicylic acid | 0.1 |
| Sodium citrate | 0.2 |
| Zinc paraphenolsulfonate | 0.2 |
| Dipotassium glycyrrhizinate | 0.1 |
| Pyridoxine hydrochloride | 0.1 |
| L-Serine | 0.1 |
| L-Menthol | 0.05 |
| Trisodium HEDTA | 0.05 |
| Powdered cellulose | 1.0 |
| Bentonite | 0.8 |
| Purified water | balance |

Preparation Example 19: gel

| (Formulation) | % by mass |
|---|---|
| Dimethyl polysiloxane | 5.0 |
| Glycerin | 2.0 |
| 1,3-Butylene glycol | 5.0 |
| 2-O-dodecylascorbic acid | 0.1 |
| 2-O-octadecylascorbic acid | 0.1 |
| Polyethylene glycol 1500 | 3.0 |
| Polyethylene glycol 20000 | 3.0 |
| Cetyl octanoate | 3.0 |
| Citric acid | 0.01 |
| Sodium citrate | 0.1 |
| Sodium hexametaphosphate | 0.1 |
| Dipotassium glycyrrhizinate | 0.1 |
| L-ascorbic acid 2-glucoside | 2.0 |
| Magnesium L-ascorbate phosphate | 0.1 |
| Tocopherol acetate | 0.1 |
| Scutellaria extract | 0.1 |
| Thime extract | 0.1 |
| Strawberry geranium extract | 0.1 |
| Trisodium edetate | 0.1 |
| Xanthan gum | 0.3 |
| Alkyl acrylate/methacrylate copolymer (PemulenTR-2) | 0.05 |
| Powdered agar | 1.5 |
| Phenoxyethanol | q.s. |
| Dibutylhydrokytoluene | q.s. |

(continued)

| (Formulation) | % by mass |
|---|---|
| Purified water | balance |

Preparation Example 20: pack

| (Formulation) | % by mass |
|---|---|
| Ethanol | 10.0 |
| 1,3-Butylene glycol | 6.0 |
| 2-O-dodecylascorbic acid | 1.0 |
| 2-O-octadecylascorbic acid | 1.0 |
| Polyethylene glycol 4000 | 2.0 |
| Olive oil | 1.0 |
| Macadamia nuts oil | 1.0 |
| Phytosteryl hydroxystearate | 0.05 |
| Lactic acid | 0.05 |
| Sodium lactate | 0.1 |
| L-Ascorbic acid disodium sulfate | 0.1 |
| α-Tocopherol 2-L-ascorbate dipotassium phosphate | 0.1 |
| Vitamin E acetate | 0.1 |
| Fish collagen | 0.1 |
| Sodium chondroitin sulfate | 0.1 |
| Sodium carboxymethylcellulose | 0.2 |
| Polyvinyl alcohol | 12.0 |
| Para-oxybenzoic acid ester | q.s. |
| Purified water | balance |
| Flavor | q.s. |

Preparation Example 21: pack

| (Formulation) | % by mass |
|---|---|
| Ethanol | 3.0 |
| Glycerin | 5.0 |
| 1,3-Butylene glycol | 6.0 |
| 2-O-octadecylascorbic acid | 1.0 |
| 2-O-hexadecylascorbic acid | 1.0 |
| Polyethylene glycol 1500 | 5.0 |
| Polyoxyethylenemethylglucoside | 2.0 |
| Glyceryl tri2-ethylhexanoate | 1.0 |
| Sodium hexametaphosphate | 0.05 |
| Hydroxypropyl-β-cyclodextrin | 0.1 |
| Dipotassium glycyrrhizinate | 0.1 |
| Loquat leaf extract | 0.1 |
| Sodium L-glutamate | 0.1 |
| Foeniculum vulgare extract | 0.1 |
| Hamamelis japonica extract | 0.1 |
| Phellodendron bark extract | 0.1 |
| Rehmannia glutinosa extract | 0.1 |
| Eucalyptus oil | 0.05 |
| Dimorpholinopyridazinone | 0.1 |
| Xanthan gum | 0.05 |
| Carboxy vinyl polymer | 0.5 |

(continued)

| (Formulation) | % by mass |
|---|---|
| Acrylic acid/alkyl methacrylate copolymer (PemulenTR-1) | 0.05 |
| Potassium hydroxide | 0.05 |
| Phenoxyethanol | q.s. |
| Purified water | balance |

Preparation Example 22: pack

| (Formulation) | % by mass |
|---|---|
| Ethanol | 3.0 |
| Glycerin | 5.0 |
| 1,3-Butylene glycol | 6.0 |
| 2-O-octadecylascorbic acid | 1.0 |
| Polyethylene glycol 1500 | 5.0 |
| Polyoxyethylenemethylglucoside | 2.0 |
| Glyceryl tri2-ethylhexanoate | 1.0 |
| Sodium hexametaphosphate | 0.05 |
| Hydroxypropyl-β-cyclodextrin | 0.1 |
| Dipotassium glycyrrhizinate | 0.1 |
| Loquat leaf extract | 0.1 |
| Sodium L-glutamate | 0.1 |
| Foeniculum vulgare extract | 0.1 |
| Hamamelis japonica extract | 0.1 |
| Phellodendron bark extract | 0.1 |
| Rehmannia glutinosa extract | 0.1 |
| Eucalyptus oil | 0.05 |
| Dimorpholinopyridazinone | 0.1 |
| Xanthan gum | 0.05 |
| Carboxy vinyl polymer | 0.5 |
| Acrylic acid/alkyl methacrylate copolymer (PemulenTR-1) | 0.05 |
| Potassium hydroxide | 0.05 |
| Phenoxyethanol | q.s. |
| Purified water | balance |

Preparation Example 23: cream

| (Formulation) | % by mass |
|---|---|
| Liquid paraffin | 3.0 |
| Vaseline | 1.0 |
| Dimethylpolysiloxane | 1.0 |
| Stearyl alcohol | 1.8 |
| Behenyl alcohol | 1.6 |
| Glycerin | 8.0 |
| Dipropylene glycol | 5.0 |
| 2-O-octadecylascorbic acid | 5.0 |
| 2-O-dodecylascorbic acid | 5.0 |
| Macadamia nuts oil | 2.0 |
| Hydrogenated oil | 3.0 |
| Squalan | 6.0 |
| Stearic acid | 2.0 |
| Cholesteryl hydroxystearate | 0.5 |

(continued)

| (Formulation) | % by mass |
|---|---|
| Cetyl 2-ethylhexanoate | 4.0 |
| Polyoxyethylene hydrogenated castor oil | 0.5 |
| Self emulsification type glycerin monostearate | 3.0 |
| Potassium hydroxide | 0.15 |
| Sodium hexametaphosphate | 0.05 |
| Trimethylglycine | 2.0 |
| α-Tocopherol 2-L-ascorbate diesterpotassium phosphate | 1.0 |
| Tocopherol acetate | 0.1 |
| Tencha extract | 0.1 |
| Paraben | q.s. |
| Trisodium edetate | 0.05 |
| 4-t-Butyl-4'-methoxybenzoylmethane | 0.05 |
| Glyceryl dipara-methoxycinnamate mono-2-ethylhexanoate | 0.05 |
| Colorant | q.s. |
| Carboxy vinyl polymer | 0.05 |
| Purified water | balance |

Preparation Example 24: cream

| (Formulation) | % by mass |
|---|---|
| Liquid paraffin | 8.0 |
| Vaseline | 3.0 |
| Dimethylpolysiloxane | 2.0 |
| Stearyl alcohol | 3.0 |
| Behenyl alcohol | 2.0 |
| Glycerin | 5.0 |
| Dipropylene glycol | 4.0 |
| 2-O-heptadecylascorbic acid | 3.0 |
| 2-O-octadecylascorbic acid | 3.0 |
| Trehalose | 1.0 |
| Pentaerythrit tetra2-ethylhexanoate | 4.0 |
| Polyoxyethyleneglyceryl monoisostearate | 2.0 |
| Polyoxyethyleneglycerin monostearate | 1.0 |
| Lipophilic type glycerin monostearate | 2.0 |
| Citric acid | 0.05 |
| Sodium citrate | 0.05 |
| Potassium hydroxide | 0.015 |
| Oil soluble Glycyrrhiza extract | 0.1 |
| Retinol palmitate (a million units) | 0.25 |
| Tocopherol acetate | 0.1 |
| Para-oxybenzoic acid ester | q.s. |
| Phenoxyethanol | q.s. |
| Dibutylhydroxytoluene | q.s. |
| Trisodium edetate | 0.05 |
| 4-t-Butyl-4'-methoxybenzoylmethane | 0.01 |
| 2-Ethylhexyl para-methoxycinnamate | 0.1 |
| β-Carotene | 0.01 |
| Polyvinyl alcohol | 0.5 |
| Hydroxyethylcellulose | 0.5 |
| Carboxy vinyl polymer | 0.05 |

(continued)

| (Formulation) | % by mass |
| --- | --- |
| Purified water | balance |
| Flavor | q.s. |

Preparation Example 25: cream

| (Formulation) | % by mass |
| --- | --- |
| Liquid paraffin | 8.0 |
| Vaseline | 3.0 |
| Dimethylpolysiloxane | 2.0 |
| Stearyl alcohol | 3.0 |
| Behenyl alcohol | 2.0 |
| Glycerin | 5.0 |
| Dipropylene glycol | 4.0 |
| 2-O-octadecylascorbic acid | 3.0 |
| Trehalose | 1.0 |
| Pentaerythrit tetra2-ethylhexanoate | 4.0 |
| Polyoxyethyleneglyceryl monoisostearate | 2.0 |
| Polyoxyethyleneglycerin monostearate | 1.0 |
| Lipophilic type glycerin monostearate | 2.0 |
| Citric acid | 0.05 |
| Sodium citrate | 0.05 |
| Potassium hydroxide | 0.015 |
| Oil soluble Glycyrrhiza extract | 0.1 |
| Retinol palmitate (a million units) | 0.25 |
| Tocopherol acetate | 0.1 |
| Para-oxybenzoic acid ester | q.s. |
| Phenoxyethanol | q.s: |
| Dibutylhydroxytoluene | q.s. |
| Trisodium edetate | 0.05 |
| 4-t-Butyl-4'-methoxybenzoylmethane | 0.01 |
| 2-Ethylhexyl para-methoxycinnamate | 0.1 |
| β-Carotene | 0.01 |
| Polyvinyl alcohol | 0.5 |
| Hydroxyethylcellulose | 0.5 |
| Carboxy vinyl polymer | 0.05 |
| Purified water | balance |
| Flavor | q.s. |

Preparation Example 26: cream

| (Formulation) | % by mass |
| --- | --- |
| Vaseline | 2.0 |
| Dimethylpolysiloxane | 2.0 |
| Ethanol | 5.0 |
| Behenyl alcohol | 0.5 |
| Batyl alcohol | 0.2 |
| Glycerin | 7.0 |
| 1,3-Butylene glycol | 5.0 |
| 2-O-dodecylascorbic acid | 1.0 |
| 2-O-octadecylascorbic acid | 1.0 |

(continued)

| (Formulation) | % by mass |
|---|---|
| Polyethylene glycol 20000 | 0.5 |
| Jojoba oil | 3.0 |
| Squalan | 2.0 |
| Phytosteryl hydroxystearate | 0.5 |
| Pentaerythrit tetra2-ethylhexanoate | 1.0 |
| Polyoxyethylene hydrogenated castor oil | 1.0 |
| Potassium hydroxide | 0.1 |
| Sodium pyrosulfite | 0.01 |
| Sodium hexametaphosphate | 0.05 |
| Stearyl glycyrrhizinate | 0.1 |
| Pantothenyl ethyl ether | 0.1 |
| Albutin | 7.0 |
| Tranexamic acid | 1.0 |
| Tocopherol acetate | 0.1 |
| Sodium hyaluronate | 0.05 |
| Para-oxybenzoic acid ester | q.s. |
| Trisodium edetate | 0.05 |
| 4-t-Butyl-4'-methoxybenzoylmethane | 0.1 |
| Mono 2-ethylhexanoate glyceryl dipara-methoxycinnamate | 0.1 |
| Yellow iron oxide | q.s. |
| Xantan gum | 0.1 |
| Carboxy vinyl polymer | 0.2 |
| Purified water | balance |

Preparation Example 27: cream

| (Formulation) | % by mass |
|---|---|
| Liquid paraffin | 10.0 |
| Dimethylpolysiloxane | 2.0 |
| Glycerin | 10.0 |
| 1,3-Butylene glycol | 2.0 |
| 2-O-dodecylascorbic acid | 1.0 |
| 2-O-octadecylascorbic acid | 1.0 |
| Erythritol | 1.0 |
| Polyethylene glycol 1500 | 5.0 |
| Squalan | 15.0 |
| Pentaerythrit tetra2-ethylhexanoate | 5.0 |
| Potassium hydroxide | 0.1 |
| Sodium hexametaphosphate | 0.05 |
| Tocopherol acetate | 0.05 |
| Para-oxybenzoic acid ester | q.s. |
| Hydroxypropylmethylcellulose | 0.3 |
| Polyvinyl alcohol | 0.1 |
| Carboxy vinyl polymer | 0.2 |
| Acrylic acid/alkyl methacrylate copolymer (PemulenTR-2) | 0.1 |
| Purified water | balance |

Preparation Example 28: cream

| (Formulation) | % by mass |
|---|---|
| Liquid paraffin | 10.0 |
| Dimethylpolysiloxane | 2.0 |
| Glycerin | 10.0 |
| 1,3-Butylene glycol | 2.0 |
| 2-O-octadecylascorbic acid | 1.0 |
| Erythritol | 1.0 |
| Polyethylene glycol 1500 | 5.0 |
| Squalan | 15.0 |
| Pentaerythrit tetra2-ethylhexanoate | 5.0 |
| Potassium hydroxide | 0.1 |
| Sodium hexametaphosphate | 0.05 |
| Tocopherol acetate | 0.05 |
| Para-oxybenzoic acid ester | q.s. |
| Hydroxypropylmethylcellulose | 0.3 |
| Polyvinyl alcohol | 0.1 |
| Carboxy vinyl polymer | 0.2 |
| Acrylic acid/alkyl methacrylate copolymer (Pemulen TR-2) | 0.1 |
| Purified water | balance |

Preparation Example 29: two-layer cream

| (Formulation) | % by mass |
|---|---|
| Dimethylpolysiloxane | 5.0 |
| Decamethylcyclopentasiloxane | 25.0 |
| Trimethylsiloxy silicate | 5.0 |
| Polyoxyethylene/methylpolysiloxane copolymer | 2.0 |
| Dipropylene glycol | 5.0 |
| 2-O-dodecylascorbic acid | 0.01 |
| 2-O-octadecylascorbic acid | 0.01 |
| Dextrin palmitate-coating particle zinc oxide (60 nm) | 15.0 |
| Dipotassium glycyrrhizinate | 0.02 |
| Glutathione | 1.0 |
| Thiotaurine | 0.05 |
| Clara extract | 1.0 |
| Paraben | q.s. |
| Phenoxyethanol | q.s. |
| Trisodium edetate | q.s. |
| 2-Ethylhexyl para-methoxycinnamate | 7.5 |
| Dimethyldistearylammonium hectright | 0.5 |
| Spherical alkyl polycrylate powder | 5.0 |
| Butylethylpropane diol | 0.5 |
| Purified water | balance |
| Flavor | q.s. |

Any injection or dermatological external agents of the preparation Examples 1-29 described above had high ADAM inhibiting effect.

## Claims

1. An ADAM inhibitor **characterized by** comprising an ascorbic acid derivative represented by the general formula (1) below:

[Formula 1]

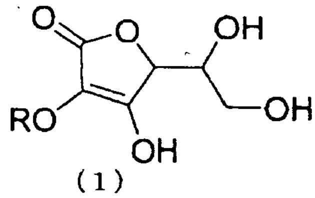

(1)

or a salt thereof wherein R represents a linear or branched alkyl group having 1 to 22 carbon atoms.

2. The ADAM inhibitor according to claim 1 **characterized in that** R is a linear or branched alkyl group having 4 to 18 carbon atoms.

3. The ADAM inhibitor according to claim 1 **characterized in that** R is a linear or branched alkyl group having 12 to 18 carbon atoms.

4. A method for inhibiting ADAM **characterized by** applying the ADAM inhibitor according to claims 1 to 3 to the skin to prevent or ameliorate the conditions caused by increase of ADAM activity.

**INTERNATIONAL SEARCH REPORT**

International application No.
PCT/JP2007/075121

A. CLASSIFICATION OF SUBJECT MATTER
*A61K31/375*(2006.01)i, *A61K8/49*(2006.01)i, *A61K8/67*(2006.01)i, *A61P17/00*(2006.01)i, *A61P17/16*(2006.01)i, *A61P25/28*(2006.01)i, *A61P29/00*(2006.01)i, *A61P35/00*(2006.01)i, *A61P43/00*(2006.01)i, *A61Q19/08*(2006.01)i,
According to International Patent Classification (IPC) or to both national classification and IPC

B. FIELDS SEARCHED
Minimum documentation searched (classification system followed by classification symbols)
A61K31/375, A61K8/49, A61K8/67, A61P17/00, A61P17/16, A61P25/28, A61P29/00, A61P35/00, A61P43/00, A61Q19/08, C07D307/62

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus(STN), EMBASE(STN), MEDLINE(STN), REGISTRY(STN), BIOSIS(STN)

C. DOCUMENTS CONSIDERED TO BE RELEVANT

| Category* | Citation of document, with indication, where appropriate, of the relevant passages | Relevant to claim No. |
|---|---|---|
| X | JP 62-226910 A (KANEBO LTD.), 05 October, 1987 (05.10.87), Particularly, Claims; page 3, upper left column, line 16 to page 6, upper left column (Family: none) | 1-3 |
| X | JP 62-221611 A (KANEBO LTD.), 29 September, 1987 (29.09.87), Particularly, Claims; page 3, line 17 to page 5, lower right column (Family: none) | 1-3 |
| X<br>Y | JP 05-058892 A (FUJIMOTO J), 09 March, 1993 (09.03.93), Particularly, Claims; page 4, right column, line 1 to page 5, right column, line 16 (Family: none) | 1-3<br>1-3 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>21 February, 2008 (21.02.08) | Date of mailing of the international search report<br>04 March, 2008 (04.03.08) |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2007/075121

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 02-235813 A (FUJIMOTO J), 18 September, 1990 (18.09.90), Particularly, Claims; page 3, upper left column, line 16 to lower right column, line 13 (Family: none) | 1-3<br>1-3 |
| X<br>Y | JP 04-128225 A (President of National Cancer Center, Takeda Chemical Industries, Ltd.), 28 April, 1992 (28.04.92), Particularly, Claims; page 3, upper left column, line 13 to page 4, lower left column, line 15 (Family: none) | 1-3<br>1-3 |
| X<br>Y | WO 96/30012 A1 (DEFEUDIS F V), 03 October, 1996 (03.10.96), Particularly, Claims; page 4, line 27 to page 6, line 15; page 14, line 21 to page 22, line 27 & AU 9653172 A | 1-3<br>1-3 |
| X<br>Y | DEFEUDIS,F.V., Excess EDRF/NO, a potentially deleterious condition that may be involved in accelerated atherogenesis and other chronic disease states, General Pharmacology, 1995, Vol.26, No.4, p.667-680 | 1-3<br>1-3 |
| Y | Shinji TERAO et al., "2-0-Octadecyl Ascorbic Acid (CV-3611): Gosei to Kassei Sanso-shu Shokyo Sayo", Vitamins, 1988, 62(11), page 631 | 1-3 |
| Y | SUNG,S. et al, Oxidative Stress Induces ADAM9 Protein Expression in Human Prostate Cancer Cells, Cancer Research, 2006, Vol.66, No.19, p.9519-9526 | 1-3 |
| Y | Hiroyuki KUBO et al., "Zenritsusen Gan Ni Okeru ADAM9 to Sanka Stress tono Kanren", The Japanese Journal of Urology, 2005, Vol.96, No.2, page 215 | 1-3 |
| Y | PIETRI,M. et al, Reactive oxygen species-dependent TNF-alpha converting enzyme activation through stimulation of 5-HT2B and alpha1D autoreceptors in neuronal cells, FASEB J, 2005, Vol.19, No.9, p.1078-1087 | 1-3 |
| Y | HINO,T. et al, Hydrogen peroxide enhances shedding of type I soluble tumor necrosis factor receptor from pulmonary epithelial cells, Am J Respir Cell Mol Biol, 1999, Vol.20, No.1, p.122-128 | 1-3 |

Form PCT/ISA/210 (continuation of second sheet) (April 2007)

**INTERNATIONAL SEARCH REPORT**

International application No.
PCT/JP2007/075121

C (Continuation). DOCUMENTS CONSIDERED TO BE RELEVANT

| Category* | Citation of document, with indication, where appropriate, of the relevant passages | Relevant to claim No. |
|---|---|---|
| Y | ZHANG,Z. et al, Reactive oxygen species mediate tumor necrosis factor alpha-converting, enzyme-dependent ectodomain shedding induced by phorbol myristate acetate, FASEB J, 2001, Vol.15, No.2, p.303-305 | 1-3 |
| Y | SANDERSON,M.P. et al, Hydrogen peroxide and endothelin-1 are novel activators of betacellulin ectodomain shedding, J Cell Biochem, 2006, Vol.99, No.2, p.609-623 | 1-3 |
| A | YANG,P. et al, The ADAMs family: coordinators of nervous system development, plasticity and repair, Prog Neurobiol, 2006, Vol.79, No.2, p.73-94 | 1-3 |

Form PCT/ISA/210 (continuation of second sheet) (April 2007)

**INTERNATIONAL SEARCH REPORT**

International application No.
PCT/JP2007/075121

Continuation of A. CLASSIFICATION OF SUBJECT MATTER
(International Patent Classification (IPC))

*C07D307/62*(2006.01)i

(According to International Patent Classification (IPC) or to both national classification and IPC)

Form PCT/ISA/210 (extra sheet) (April 2007)

**INTERNATIONAL SEARCH REPORT**

International application No.
PCT/JP2007/075121

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 4
because they relate to subject matter not required to be searched by this Authority, namely:
Claim 4 pertains to methods for treatment of the human body by therapy and thus relates to a subject matter which this International Searching Authority is not required, under the provisions of the PCT Rule 39.1(iv), to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Yang P ; Baker KA ; Hagg T.** The ADAMs family: Coordinators of nervous system development, plasticity and repair. *Prog Neurobiol,* 2006, vol. 79, 73-94
- **Arribas J ; Bech-Serra JJ ; Santiago-Josefat B.** ADAMs, cell migration and cancer. *Cancer Metastasis Rev,* 2006, vol. 25, 57-68
- **Kodama T ; Ikeda E ; Okada A et al.** ADAM12 is selectively overexpressed in human glioblastomas and is associated with glioblastoma cell proliferation and shedding of heparin-binding epidermal growth factor. *Am J Pathol,* 2004, vol. 165, 1743-53
- **Liu PC ; Liu X ; Li Y et al.** Identification of ADAM10 as a Major Source of HER2 Ectodomain Sheddase Activity in HER2 Overexpressing Breast Cancer Cells. *Cancer Biol Ther,* 2006, vol. 5, 657-64
- **Rocks N ; Paulissen G ; Quesada Calvo F et al.** Expression of a disintegrin and metalloprotease (ADAM and ADAMTS) enzymes in human non-small-cell lung carcinomas (NSCLC). *Br J Cancer,* 2006, vol. 94, 724-30
- **Asakura, M. et al.** Cardiac hypertrophy is inhibited by antagonism of ADAM12 processing of HB-EGF: metalloproteinase inhibitors as a new therapy. *Nat. Med.,* 2002, vol. 8, 35-40